# EUROPEAN PATENT APPLICATION

(11) **EP 4 538 360 A1**
(43) Date of publication of application: **16.04.2025**
(21) Application number: 22793500.4
(22) Date of filing: 15.07.2022
(51) Int. Cl.: C12M 1/34, C12M 1/00, B01L 3/00

(54) **MICROFLUIDIC CHIP DETECTION CARD BOX**

(30) Priority: 06.06.2022 CN 202210633420; 06.06.2022 CN 202221409066 U
(71) Applicant: Daan Gene Co., Ltd., Guangzhou, Guangdong 510665 (CN)
(72) Inventor: JIANG, Xiwen, Guangzhou, Guangdong 510665 (CN); LIU, Linbo, Guangzhou, Guangdong 510665 (CN); SHU, Haitao, Guangzhou, Guangdong 510665 (CN); LI, Junjie, Guangzhou, Guangdong 510665 (CN)
(74) Representative: Handsome I.P. Ltd
(86) International application number: PCT/CN2022/106128
(87) International publication number: WO 2023/236313

(57) **Abstract**

The present disclosure pertains to the technical field of nucleic acid detection, and relates to a microfluidic chip-equipped detection cassette. The microfluidic chip-equipped detection cassette includes a housing, provided with a functional chamber, a first flow channel assembly, a second flow channel assembly, and a result display assembly, wherein the first flow channel assembly and the second flow channel assembly are both in communication with the functional chamber, the functional chamber is in communication with the result display assembly, and the result display assembly is configured to display a detection result. In the microfluidic chip-equipped detection cassette, sample dispensing for nucleic acid amplification and the diluent dispensing are carried out in different flow channels, less reagent is consumed, and thus waste of the reagent is mitigated; and sample dispensing for nucleic acid amplification and the diluent dispensing are carried out separately via the first flow channel assembly and the second flow channel assembly, such that sample dispensing and detection are accurately controlled, and the advantages of simple operation, high security and reliability, high detection efficiency, high sensitivity, less sample consumption and the like are achieved. By sample dispensing for nucleic acid amplification, a variety of different biological samples are detected, and the application scope is extensive.

## Description

This application is based upon and claims priorities to Chinese Patent Application No. 202210633420.8, filed June 6, 2022 and entitled "MICROFLUIDIC CHIP-EQUIPPED DETECTION CASSETTE," and Chinese Patent Application No. 202221409066.2, filed on June 6, 2022 and entitled "MICROFLUIDIC CHIP-EQUIPPED DETECTION CASSETTE," the disclosures of which are incorporated herein by reference in their entireties.

### TECHNICAL FIELD

The present disclosure relates to the technical field of nucleic acid detection, and in particular, relates to a microfluidic chip-equipped detection cassette.

### BACKGROUND

With the development of science and technology, molecular diagnostic technology has been widely used in epidemiological investigation, infectious disease diagnosis, food hygiene inspection and other aspects, and a series of detection instruments, such as an isothermal amplifier and a PCR amplifier, have gradually replaced the traditional way of smear or microscopy.

At present, the steps of nucleic acid extraction and purification, nucleic acid amplification, and nucleic acid molecular hybridization applied to commercial diagnosis are performed separately, and such instruments as a sample preparation instrument for extracting nucleic acid, a nucleic acid extraction instrument, and a PCR amplifier for amplification need to be operated separately. The specific process for completing the whole nucleic acid detection mainly includes the following steps: manually extracting a sample or manually add a sample into a fully-automatic nucleic acid extractor to extract and purify a nucleic acid, manually transferring a solution of the purified nucleic acid to a nucleic acid amplifier, amplifying the nucleic acid using the nucleic acid amplifier, and finally transferring an amplified product to the fully-automatic nucleic acid extractor for analysis. For nucleic acid detection using largescale instruments, the device required for the whole detection process is complicated, bulky, less efficient in detection, and poor in flexibility. In addition, the expensive instruments and devices are expensive, the whole operation process is complicated, the operations need to be performed by highly professional personnel, and the detection needs to be operated by skilled technicians. As a result, portable detection at home or other places cannot be achieved.

### SUMMARY

It is an object of the present disclosure to provide a microfluidic chip-equipped detection cassette, which solves the technical problems of complicated devices, large size, low detection efficiency, poor flexibility, complicated operation process and inability to realize portable detection at home or other places in the existing nucleic acid detection process.

In order to solve the above problem, embodiments of the present disclosure provide the following technical solutions.

A microfluidic chip-equipped detection cassette includes a housing, provided with a functional chamber, a first flow channel assembly, a second flow channel assembly, and a result display assembly, wherein the first flow channel assembly and the second flow channel assembly are both in communication with the functional chamber, the functional chamber is in communication with the result display assembly, and the result display assembly is configured to display a detection result.

Further, the housing includes a first housing, a second housing, a cover plate, and a flow channel interlayer, wherein the first housing is arranged on the second housing, the flow channel interlayer is arranged on the first housing, the cover plate is arranged on the flow channel interlayer, the functional chamber is arranged in the first housing and the flow channel interlayer, the first flow channel assembly is arranged on the flow channel interlayer, the second flow channel assembly is arranged on the cover plate, and the result display assembly is arranged on the cover plate and the flow channel interlayer.

Further, the first flow channel assembly includes a first piercing structure, an aligning structure, a fluid inlet, and a first microflow channel, wherein the first piercing structure and the aligning structure are arranged on a surface, facing away from the first housing, of the flow channel interlayer, the aligning structure is provided with a positioning slot, the first piercing structure is arranged in the aligning structure, the fluid inlet runs through the flow channel interlayer and is arranged in the first piercing structure, the fluid inlet is in communication with the first microflow channel, the first microflow channel is arranged on a surface, facing towards the first housing, of the flow channel interlayer, and the first microflow channel is in communication with the functional chamber.

Further, the second flow channel assembly includes a second piercing structure, a well, and a second microflow channel, wherein the second piercing structure is arranged on a surface, facing away from the flow channel interlayer, of the cover plate, the well runs through the cover plate and is arranged in the second piercing structure, the well is in communication with the second microflow channel, the second microflow channel is arranged on a surface, facing towards the flow channel interlayer, of the cover plate, the second microflow channel is in communication with the functional chamber, the cover plate is provided with an intake hole, and the first piercing structure is arranged in the intake hole.

Further, a plurality of functional chambers are provided, and a connection branch is arranged at one end, close to the functional chamber, of each of the first microflow channel and the second microflow channel, wherein a number of the connection branches is equal to a number of the functional chambers and the connection branches are respectively in communication with the functional chamber.

Further, the result display assembly includes a detection kit, wherein the detection kit includes a chromatographic strip and/or a visible dye detection strip and/or an optical detection instrument and/or an electrical signal detection instrument.

Further, the result display assembly further includes a strip slot and an observation window, wherein the strip slot is opened in a surface, facing away from the first housing, of the flow channel interlayer, the observation window is arranged at a position corresponding to the strip slot on the cover plate, and the flow channel interlayer is provided with a fluid buffering tank, two ends of the fluid buffering tank being respectively in communication with the functional chamber and the strip slot.

Further, each of the first microflow channel and the second microflow channel has a width of 0.005 to 50 mm and a depth of 0.005 to 50 mm.

Further, the first housing is provided with a first latch and a positioning hole, and the second housing is provided with a latching recess and a positioning post, wherein the positioning hole is engaged with the positioning post, and the first latch is engaged with the latching recess; and

a mounting slot is arranged at a position of the functional chamber on the first housing, and a fixing slot engaged with the mounting slot is arranged on the second housing, wherein an upper end of the fixing slot is mounted in the mounting slot, and a heat preservation cotton is arranged in the fixing slot.

Further, the microfluidic chip-equipped detection cassette further includes a heating assembly, a temperature sensor, and a PCB, wherein the heating assembly includes a heating module and a temperature control module, wherein the heating module is arranged on a surface, facing towards the second housing, of the first housing, the heating module is opposite to the functional chamber, the temperature control module and the PCB are arranged on the first housing, the temperature sensor is arranged in the functional chamber, and the heating assembly and the temperature sensor are electrically connected to the PCB; and
wherein the first housing is provided with a second latch and a positioning structure, and the PCB is mounted on the first housing by the second latch and the positioning structure.

Further, the functional chamber includes a first chamber and a second chamber connected to the first chamber, wherein the first chamber is arranged in the first housing, and the second chamber is arranged in the flow channel interlayer.

Further, the first chamber and the second chamber are connected by laser welding, ultrasonic welding, thermocompression bonding, plasma bonding, solvent bonding, or adhesive bonding; and
a sealing structure is formed between the cover plate and the flow channel interlayer, and the cover plate and the flow channel interlayer are connected by laser welding, ultrasonic welding, thermocompression bonding, plasma bonding, solvent bonding, or adhesive bonding.

Compared with the related art, the embodiments of the present disclosure mainly achieve the following beneficial effects:
In the microfluidic chip-equipped detection cassette according to the embodiments of the present disclosure, the first flow channel assembly and the second flow channel assembly are respectively configured to dispense a sample for nucleic acid amplification and dispense a diluent. A reaction liquid for nucleic acid amplification flows into the functional chamber via the first flow channel assembly, and the reaction solution for nucleic acid amplification is reacted with a reaction reagent pre-loaded in the functional chamber. The diluent flows into the functional chamber via the second flow channel assembly, after the functional chamber is fully filled with the liquid, the liquid flows to the result display assembly, and a biochemical reaction result is checked via the result display assembly. In the microfluidic chip-equipped detection cassette, sample dispensing for nucleic acid amplification and the diluent dispensing are carried out in different flow channels, less reagent is consumed, and thus waste of the reagent is mitigated. In the microfluidic chip-equipped detection cassette, sample dispensing for nucleic acid amplification and the diluent dispensing are carried out separately via the first flow channel assembly and the second flow channel assembly, such that sample dispensing and detection are accurately controlled, and the advantages of simple operation, high security and reliability, high detection efficiency, high sensitivity, less sample consumption and the like are achieved. By sample dispensing for nucleic acid amplification, a variety of different biological samples are detected, and the application scope is extensive.

### BRIEF DESCRIPTION OF THE DRAWINGS

For clearer descriptions of technical solutions according to the embodiments of the present disclosure, drawings that are to be referred for description of the embodiments are briefly described hereinafter. Apparently, the drawings described hereinafter merely illustrate some embodiments of the present disclosure. Persons of ordinary skill in the art may also derive other drawings based on the drawings described herein without any creative effort.
FIG. 1 is a schematic structural view of a microfluidic chip-equipped detection cassette according to some embodiments of the present disclosure;
FIG. 2 is a schematic structural view of a cover plate according to some embodiments of the present disclosure;
FIG. 3 is a schematic structural view of a flow channel interlayer according to some embodiments of the present disclosure;
FIG. 4 is a schematic structural view, taken from another angle of view, of the flow channel interlayer according to some embodiments of the present disclosure;
FIG. 5 is a schematic structural view of a first housing according to some embodiments of the present disclosure; and
FIG. 6 is a schematic structural view of a second housing according to some embodiments of the present disclosure.

Reference numerals and denotations thereof:
1-housing; 11-first housing; 111-first latch; 112-positioning hole; 113-mounting slot; 114-USB port; 115-second latch; 116-positioning structure; 12-second housing; 121-latching recess; 122-positioning post; 123-fixing slot; 13-cover plate; 131-intake hole; 14- flow channel interlayer; 141-fluid buffering tank; 2-functional chamber; 3-first flow channel assembly; 31-first piercing structure; 32-aligning structure; 321-positioning slot; 33-fluid inlet; 34-first microflow channel; 4-second flow channel assembly; 41-second piercing structure; 42-well; 5-result display assembly; 51-detection strip; 52-observation window; 53- detection line; 6-heating module; and 7-temperature control module.

### DETAILED DESCRIPTION

Unless otherwise defined, all the technical and scientific terms used in this specification are the same as those usually understood by persons skilled in the art of the present disclosure. Additionally, the terms used in the specification of the present disclosure are merely for description the embodiments of the present disclosure, but are not intended to limit the present disclosure. The terms "comprise," "include," and variations thereof in the specification, claims and accompanying drawings are intended to define a non-exclusive meaning. The terms such as "first," "second," and the like in the specifications, claims or the accompanying drawings of the present disclosure are intended to distinguishing different objects but are not intended to define a specific sequence.

The term "embodiments" in this specification signifies that the specific feature, structure or feature described with reference to the embodiments may be covered in at least one embodiment of the present disclosure. This term, when appearing in various positions of the specification, neither indicates the same embodiment, nor indicates an independent or optional embodiment that is exclusive of the other embodiments. A person skilled in the art would implicitly or explicitly understand that the embodiments described in this specification may be incorporated with other embodiments.

To make a person skilled in the art better understand the technical solutions of the present disclosure, the technical solutions according to the embodiments of the present disclosure are clearly and completely described with reference to the accompanying drawings.

### Embodiments

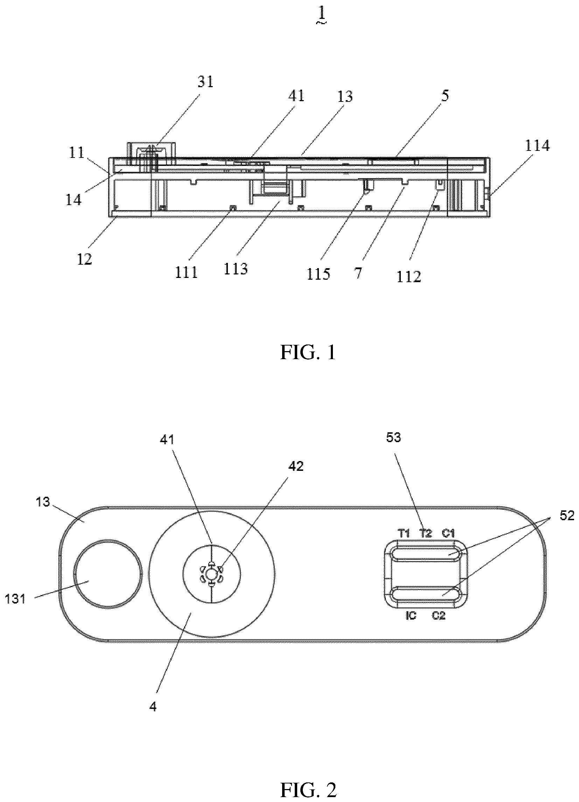

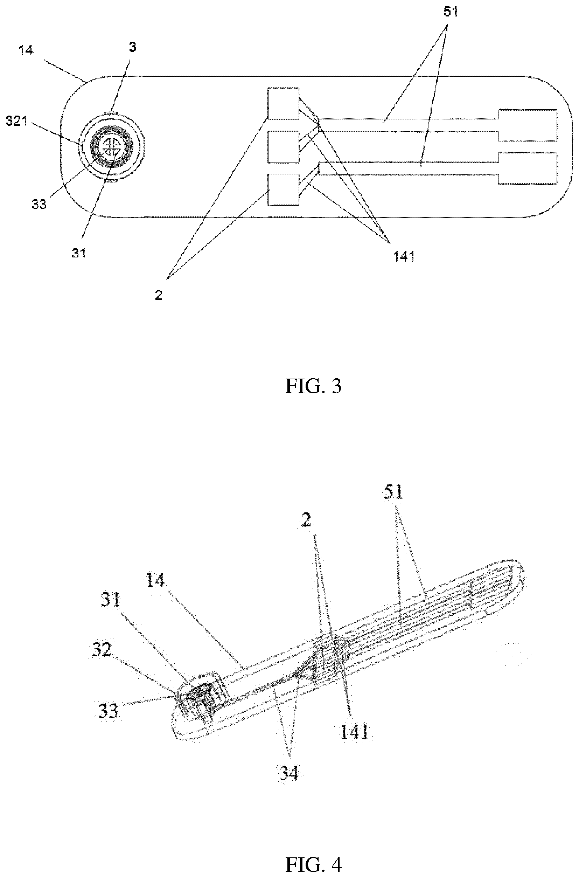

As illustrated in FIG. 1 to FIG. 3, a microfluidic chip-equipped detection cassette includes a housing 1, provided with a functional chamber 2, a first flow channel assembly 3, a second flow channel assembly 4, and a result display assembly 5. The first flow channel assembly 3 and the second flow channel assembly 4 are both in communication with the functional chamber 2. The functional chamber 2 is in communication with the result display assembly 5. The result display assembly 5 is configured to display a detection result.

In the microfluidic chip-equipped detection cassette according to the embodiments of the present disclosure, the first flow channel assembly 3 and the second flow channel assembly 4 are respectively configured to dispense a sample for nucleic acid amplification and dispense a diluent. A reaction liquid for nucleic acid amplification flows into the functional chamber 2 via the first flow channel assembly 3, and the reaction solution for nucleic acid amplification is reacted with a reaction reagent pre-loaded in the functional chamber 2. The diluent flows into the functional chamber 2 via the second flow channel assembly 4, after the functional chamber 2 is fully filled with the liquid, the liquid flows to the result display assembly 5, and a biochemical reaction result is checked via the result display assembly 5. In the microfluidic chip-equipped detection cassette, sample dispensing for nucleic acid amplification and the diluent dispensing are carried out in different flow channels, less reagent is consumed, and thus waste of the reagent is mitigated. In the microfluidic chip-equipped detection cassette, sample dispensing for nucleic acid amplification and the diluent dispensing are carried out separately via the first flow channel assembly 3 and the second flow channel assembly 4, such that sample dispensing and detection are accurately controlled, and the advantages of simple operation, high security and reliability, high detection efficiency, high sensitivity, less sample consumption and the like are achieved. By sample dispensing for nucleic acid amplification, a variety of different biological samples are detected, and the application scope is extensive.

It should be noted that the functional chamber 2 is a chamber in which the biochemical reaction of the detection reagent occurs, and is not limited by the type of detection and the object of detection; the pre-loaded reaction reagent in the functional chamber 2 refers to the reagent material used in the biochemical detection, which is usually pre-loaded in the functional chamber 2. According to different detection targets, the components contained in the material and the preloading method can be determined in various ways according to the environment required for the material.

The housing 1 includes a first housing 111, a second housing 121, a cover plate 13, and a flow channel interlayer 14. The first housing 111 is arranged on the second housing 121.

In some embodiments, the flow channel interlayer 14 is arranged on the first housing 111, the cover plate 13 is arranged on the flow channel interlayer 14, the functional chamber 2 is arranged on the first housing 111 and the flow channel interlayer 14, the first flow channel assembly 3 is arranged on the flow channel interlayer 14, the second flow channel assembly 4 is arranged on the cover plate 13, and the result display assembly 5 is arranged on the cover plate 13 and the flow channel interlayer 14.

The result display assembly 5 includes a detection kit. The detection kit includes a chromatographic strip and/or a visible dye detection strip and/or an optical detection instrument and/or an electrical signal detection instrument. Different detection methods can be designed according to requirements, and nucleic acid detection methods include hybridization fluorescence, isothermal amplification, real-time fluorescence PCR, high-resolution melting curve, electrochemical nucleic acid aptamer, and the like. It is to be understood that the corresponding detection member may be designed according to the detection method, and either the detection method or the detection apparatus is not limited as long as the product showing the result of the reagent after the reaction is suitable for the present disclosure.

In some embodiments, the result display assembly 5 further includes a strip slot 51 and an observation window 52. The strip slot 51 is opened in a surface, facing away from the first housing 111, of the flow channel interlayer 14, the observation window 52 is arranged at a position corresponding to the strip slot 51 on the cover plate 13, and the flow channel interlayer 14 is provided with a fluid buffering tank 141. Two ends of the fluid buffering tank 141 are respectively in communication with the functional chamber 2 and the strip slot 51.

In some embodiments, the detection element includes a chromatographic test paper, wherein the chromatographic test paper is used for presenting the detection result, the stripshaped fiber chromatographic material with a detection line 53 and a quality control line immobilized thereon is used as a stationary phase, the test solution is used as a mobile phase, a fluorescently labeled antibody or antigen is immobilized on a connection pad, and the analyte is moved and captured on the chromatographic strip by capillary action so as to perform detection; the chromatographic strip is pre-mounted in the strip slot 51, the test strip slot 51 limits and fixes the front, rear, left and right of the chromatographic strip, and the cover plate 13 and the flow channel interlayer 14 cooperate to press the upper surface of the chromatographic strip, so as to fix the chromatographic strip.

In some embodiments, the observation window 52 is opened in an upper surface of the cover plate 13, the cover plate 13 is made of a transparent material, and upon completion of assembling of the microfluidic chip-equipped detection cassette, the observation window 52 is above the strip slot 51 and one-to-one corresponds to a color region of the chromatographic strip. In nucleic acid detection using the microfluidic chip-equipped detection cassette, a detection result may be observed via the observation window 52. Upon completion of reaction, the chromatographic strip changes color, and whether the detection result is positive or negative by checking the color changes.

In some embodiments, the functional chamber 2 includes a first chamber and a second chamber connected to the first chamber. The first chamber is arranged in the first housing 111, and the second chamber is arranged in the flow channel interlayer 14. The functional chamber 2 is composed of a first chamber and a second chamber. The first chamber is arranged on the first housing 111, and the second chamber is arranged on the flow channel interlayer 14. The first chamber and the second chamber are engaged and aligned with and fixed to each other. The fixation may be achieved by laser welding, ultrasonic welding, thermocompression bonding, plasma bonding, solvent bonding, or adhesive bonding.

As illustrated in FIG. 1, FIG. 3, and FIG. 4, the first flow channel assembly 3 includes a first piercing structure 31, an aligning structure 32, a fluid inlet 33, and a first microflow channel 34. The first piercing structure 31 and the aligning structure 32 are arranged on a surface, facing away from the first housing 111, of the flow channel interlayer 14, the aligning structure 32 is provided with a positioning slot 321, the first piercing structure 31 is arranged in the aligning structure 32, the fluid inlet 33 runs through the flow channel interlayer 14 and is arranged in the first piercing structure 31, the fluid inlet 33 is in communication with the first microflow channel 34, the first microflow channel 34 is arranged on a surface, facing towards the first housing 111, of the flow channel interlayer 14, and the first microflow channel 34 is in communication with the functional chamber 2.

In some embodiments, a plurality of functional chambers 2 are provided, and a connection branch is arranged at one end, close to the functional chamber 2, of the first microflow channel 34. A number of the connection branches is equal to a number of the functional chambers 2 and the connection branches are respectively in communication with the functional chambers 2. Exemplarily, three functional chambers 2 are provided, and likewise three connection branches are provided for the first microflow channel 34.

The flow channel interlayer 14 is provided with a first piercing structure 31, an aligning structure 32, a fluid inlet 33, and a first microflow channel 34. A protrusion on a reagent tube sealed by an aluminum film is aligned with the aligning structure 32 and inserted, and the piercing structure pierces the aluminum film. In this way, under the effect of pressure, the liquid flows out and flows into the first microflow channel 34 on the flow channel interlayer 14 via the fluid inlet 33, the liquid flows into the functional chamber 2 via three connection branches, and the liquid is capable of being evenly divided into three branches and flowing into the functional chamber 2. Finally, the liquid is reacted with the pre-loaded reagent in the functional chamber 2. Optionally, the liquid is the reaction solution for nucleic acid amplification.

As illustrated in FIG. 1 and FIG. 2, the second flow channel assembly 4 includes a second piercing structure 41, a well 42, and a second microflow channel. The second piercing structure 41 is arranged on a surface, facing away from the flow channel interlayer 14, of the cover plate 13, the well 42 runs through the cover plate 13 and is arranged in the second piercing structure 41, the well 42 is in communication with the second microflow channel, the second microflow channel is arranged on a surface, facing towards the flow channel interlayer 14, of the cover plate 13, the second microflow channel is in communication with the functional chamber 2, the cover plate 13 is provided with an intake hole 131, and the first piercing structure 31 is arranged in the intake hole 131.

In some embodiments, a connection branch is arranged at one end, close to the functional chamber 2, of the second microflow channel. A number of the connection branches is equal to a number of the functional chambers 2 and the connection branches are respectively in communication with the functional chambers 2. Exemplarily, three functional chambers 2 are provided, and likewise three connection branches are provided for the second microflow channel.

The cover plate 13 is provided with a second piercing structure 41, a well 42, and a second microflow channel. A blister package loaded with liquid is pressed, the blister package is pierced, the liquid flows out and flows into the functional chamber 2 via the well 42 over the second microflow channel on a lower surface of the cover plate 13, the liquid evenly flows into the functional chamber 2 via three connection branches, and the liquid is capable of being divided into three branches and flowing into the functional chamber 2. After the functional chamber 2 is fully filled with the liquid, the liquid flows to the chromatographic strip in the strip slot 51. Upon completion of reaction, the chromatographic strip changes color, a biochemical reaction result is checked via the result display assembly 5, and whether the detection result is positive or negative by checking the color changes. Optionally, the liquid the diluent.

In some embodiments, each of the first microflow channel 34 and the second microflow channel has a width of 0.005 to 50 mm and a depth of 0.005 to 50 mm. It is understood that the widths of the first microflow channel 34 and the second microflow channel may be 0.005 mm, 0.05 mm, 0.5 mm, 5 mm, 50 mm, or any value between each adjacent two of these values; and likewise, the depths of the first microflow channel 34 and the second microflow channel may be 0.005 mm, 0.05 mm, 0.5 mm, 5 mm, 50 mm, or any value between each adjacent two of these values.

Preferably, each of the first microflow channel 34 and the second microflow channel has a width of 0.01 to 10 mm and a depth of 0.01 to 10 mm. It is understood that the widths of the first microflow channel 34 and the second microflow channel may be 0.01 mm, 0.5 mm, 0.1 mm, 0.5 mm, 1 mm, 5 mm, 10 mm, or any value between each adjacent two of these values; and likewise, the depths of the first microflow channel 34 and the second microflow channel may be 0.01 mm, 0.05 mm, 0.1 mm, 0.5 mm, 1 mm, 5 mm, 10 mm, or any value between each adjacent two of these values.

In some embodiments, the first microflow channel 34 and the second microflow channel are fabricated by machining over machine tool, laser ablation, 3D printing, injection molding, or chemical etching machining.

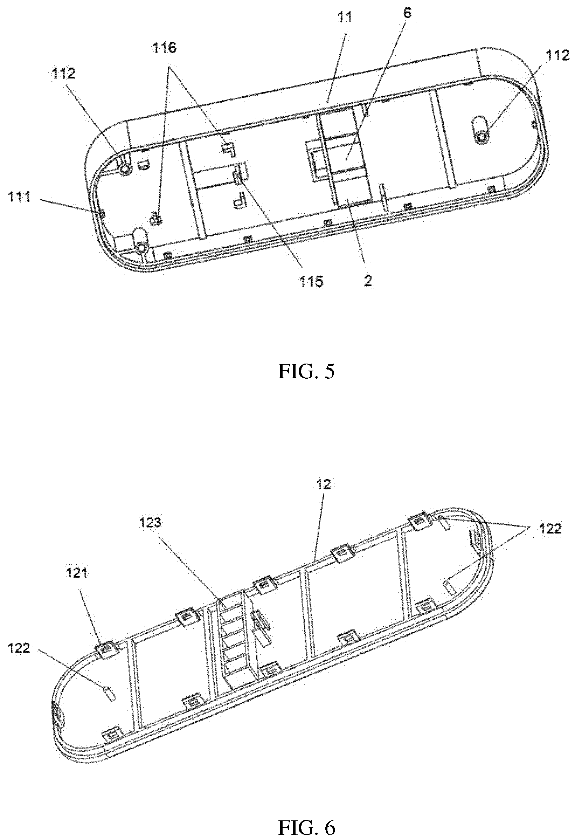

As illustrated in FIG. 1, FIG. 5, and FIG. 6, the first housing 111 is provided with a first latch 111 and a positioning hole 112, and the second housing 121 is provided with a latching recess 121 and a positioning post 122. The positioning hole 112 is engaged with the positioning post 122, and the first latch 111 is engaged with the latching recess 121. The positioning hole 112 and the positioning post 122 are engaged with each other to facilitate assembling, and the latch and the latching recess 121 are engaged with each other to form a dead lock, such that the first housing 111 and the second housing 121 are fixed and assembled.

A mounting slot 113 is arranged at a position of the functional chamber 2 on the first housing 111, and a fixing slot 123 engaged with the mounting slot 113 is arranged on the second housing 121. An upper end of the fixing slot 123 is mounted in the mounting slot 113, and a heat preservation cotton is arranged in the fixing slot 123. In this way, a temperature preservation effect is ensured for the function chamber 2.

The microfluidic chip-equipped detection cassette further includes a heating assembly, a temperature sensor, and a PCB. The heating assembly includes a heating module 6 and a temperature control module 7. The heating module 6 is arranged on a surface, facing towards the second housing 121, of the first housing 111, the heating module 6 is opposite to the functional chamber 2, the temperature control module 7 and the PCB are arranged on the first housing 111, the temperature sensor is arranged in the functional chamber 2, and the heating assembly and the temperature sensor are electrically connected to the PCB; and optionally, the temperature sensor is an NTC sensor, the temperature control module 7 supplies power to the heating module 6 by a built-in battery or the USB port, wherein upon power on, an internal indicator is lit and the heating module 6 starts operating and generating heat, and when the NTC sensor senses a temperature desired for the reaction, a program enters heat preservation, and a biochemical reaction is carried out in the functional chamber 2. The first housing 111 is provided with a USB port 114, and a progress of a detection reaction is controlled by connecting or disconnecting a UBS line or controlled by turning on or turning off a power switch.

The first housing 111 is provided with a second latch 115 and a positioning structure 116, and the PCB is mounted on the first housing 111 by the second latch 115 and the positioning structure 116.

A sealing structure is formed by lamination between the cover plate 13 and the flow channel interlayer 14, and the cover plate 13 and the flow channel interlayer 14 are connected by laser welding, ultrasonic welding, thermocompression bonding, plasma bonding, solvent bonding, or adhesive bonding. The nucleic acid amplification and detection process is completely blocked from the external environment, and thus no aerosol contamination is caused and no contamination is caused to the external environment and device.

Compared with the related art, the microfluidic chip-equipped cassette according to the embodiments of the present disclosure integrates the nucleic acid amplification reactions together, achieves precise control of sample dispensing and detection by microfluidic, imposes low professional requirements on the operation process, does not require professional training and learning for detection operation and can be operated by hand, is easy to operate, has high detection efficiency and high sensitivity, is secure and reliable, is capable of quickly obtaining a detection report, is capable of obtaining an accurate detection result without expensive largescale instruments, and greatly reduces the detection cost. When product is improperly operated, no harmful consequences are caused, and the product has high safety and is very suitable for the home self-inspection of common people. At the same time, the microfluidic chip-equipped cassette is not limited in terms of detection site, and the detection does not need to be performed in a fixed laboratory. The microfluidic chip-equipped cassette is portable and convenient, and is applicable to community detection or home self-detection. The microfluidic chip-equipped cassette is capable of detecting nasal swabs, throat swabs and other samples, and has a wide range of application. Through home self-detection in combination with appropriate measures, the anxiety of people is alleviated and unnecessary medical resource occupation and crowd aggregation are prevented.

It is apparent that the embodiments described above are only exemplary ones, but not all embodiments of the present disclosure, and that the attached drawings illustrate exemplary embodiments of the present disclosure but do not limit the scope of the present disclosure. The present disclosure may be embodied in many different forms and, on the contrary, these embodiments are provided for thorough and complete understanding of the present disclosure. Although the present disclosure has been described in detail with reference to the above embodiments, those skilled in the art will be able to make modifications to the technical solutions disclosed in the specific embodiments or make equivalent substitutions for some of the technical features. Any equivalent structure made based on the specification and accompanying drawings of the present disclosure, even if being directly or indirectly applied to some other related technical fields, shall all fall within the protection scope of the present disclosure.

## Claims

1. A microfluidic chip-equipped detection cassette, comprising:
a housing, provided with a functional chamber, a first flow channel assembly, a second flow channel assembly, and a result display assembly, wherein the first flow channel assembly and the second flow channel assembly are both in communication with the functional chamber, the functional chamber is in communication with the result display assembly, and the result display assembly is configured to display a detection result.

2. The microfluidic chip-equipped detection cassette according to claim 1, wherein
the housing comprises a first housing, a second housing, a cover plate, and a flow channel interlayer, wherein the first housing is arranged on the second housing, the flow channel interlayer is arranged on the first housing, the cover plate is arranged on the flow channel interlayer, the functional chamber is arranged in the first housing and the flow channel interlayer, the first flow channel assembly is arranged on the flow channel interlayer, the second flow channel assembly is arranged on the cover plate, and the result display assembly is arranged on the cover plate and the flow channel interlayer.

3. The microfluidic chip-equipped detection cassette according to claim 2, wherein
the first flow channel assembly comprises a first piercing structure, an aligning structure, a fluid inlet, and a first microflow channel, wherein the first piercing structure and the aligning structure are arranged on a surface, facing away from the first housing, of the flow channel interlayer, the aligning structure is provided with a positioning slot, the first piercing structure is arranged in the aligning structure, the fluid inlet runs through the flow channel interlayer and is arranged in the first piercing structure, the fluid inlet is in communication with the first microflow channel, the first microflow channel is arranged on a surface, facing towards the first housing, of the flow channel interlayer, and the first microflow channel is in communication with the functional chamber.

4. The microfluidic chip-equipped detection cassette according to claim 3, wherein
the second flow channel assembly comprises a second piercing structure, a well, and a second microflow channel, wherein the second piercing structure is arranged on a surface, facing away from the flow channel interlayer, of the cover plate, the well runs through the cover plate and is arranged in the second piercing structure, the well is in communication with the second microflow channel, the second microflow channel is arranged on a surface, facing towards the flow channel interlayer, of the cover plate, the second microflow channel is in communication with the functional chamber, the cover plate is provided with an intake hole, and the first piercing structure is arranged in the intake hole.

5. The microfluidic chip-equipped detection cassette according to claim 4, wherein
a plurality of functional chambers are provided, and a connection branch is arranged at one end, close to the functional chamber, of each of the first microflow channel and the second microflow channel, wherein a number of the connection branches is equal to a number of the functional chambers and the connection branches are respectively in communication with the functional chambers.

6. The microfluidic chip-equipped detection cassette according to claim 2, wherein
the result display assembly comprises a detection kit, wherein the detection kit comprises a chromatographic strip and/or a visible dye detection strip and/or an optical detection instrument and/or an electrical signal detection instrument.

7. The microfluidic chip-equipped detection cassette according to claim 6, wherein
the result display assembly further comprises a strip slot and an observation window, wherein the strip slot is opened in a surface, facing away from the first housing, of the flow channel interlayer, the observation window is arranged at a position corresponding to the strip slot on the cover plate, and the flow channel interlayer is provided with a fluid buffering tank, two ends of the fluid buffering tank being respectively in communication with the functional chamber and the strip slot.

8. The microfluidic chip-equipped detection cassette according to claim 4, wherein
each of the first microflow channel and the second microflow channel has a width of 0.005 to 50 mm and a depth of 0.005 to 50 mm.

9. The microfluidic chip-equipped detection cassette according to claim 2, wherein
the first housing is provided with a first latch and a positioning hole, and the second housing is provided with a latching recess and a positioning post, wherein the positioning hole is engaged with the positioning post, and the first latch is engaged with the latching recess; and
a mounting slot is arranged at a position of the functional chamber on the first housing, and a fixing slot engaged with the mounting slot is arranged on the second housing, wherein an upper end of the fixing slot is mounted in the mounting slot, and a heat preservation cotton is arranged in the fixing slot.

10. The microfluidic chip-equipped detection cassette according to claim 2, further comprising:
a heating assembly, a temperature sensor, and a PCB, wherein the heating assembly comprises a heating module and a temperature control module, wherein the heating module is arranged on a surface, facing towards the second housing, of the first housing, the heating module is opposite to the functional chamber, the temperature control module and the PCB are arranged on the first housing, the temperature sensor is arranged in the functional chamber, and the heating assembly and the temperature sensor are electrically connected to the PCB; and
wherein the first housing is provided with a second latch and a positioning structure, and the PCB is mounted on the first housing by the second latch and the positioning structure.

11. The microfluidic chip-equipped detection cassette according to claim 2, wherein
the functional chamber comprises a first chamber and a second chamber connected to the first chamber, wherein the first chamber is arranged in the first housing, and the second chamber is arranged in the flow channel interlayer.

12. The microfluidic chip-equipped detection cassette according to claim 11, wherein
the first chamber and the second chamber are connected by laser welding, ultrasonic welding, thermocompression bonding, plasma bonding, solvent bonding, or adhesive bonding; and
a sealing structure is formed between the cover plate and the flow channel interlayer, and the cover plate and the flow channel interlayer are connected by laser welding, ultrasonic welding, thermocompression bonding, plasma bonding, solvent bonding, or adhesive bonding.

13. The microfluidic chip-equipped detection cassette according to claim 7, wherein
the detection kit comprises the chromatographic strip, wherein the chromatographic strip is configured to present a display result, the chromatographic strip is pre-mounted in the strip slot, the strip slot limits and fixes the chromatographic strip by four sides, and the cover plate and the flow channel interlayer are engaged with each other to press an upper surface of the chromatographic strip to fix the chromatographic strip.

14. The microfluidic chip-equipped detection cassette according to claim 13, wherein
the observation window is opened in an upper surface of the cover plate, the cover plate is made of a transparent material, and upon completion of assembling of the microfluidic chip-equipped detection cassette, the observation window is above the strip slot and one-to-one corresponds to a color region of the chromatographic strip.

15. The microfluidic chip-equipped detection cassette according to claim 5, wherein
three functional chambers are provided, and three connection branches are provided for each of the first microflow channel and the second microflow channel.

16. The microfluidic chip-equipped detection cassette according to claim 11, wherein
the first microflow channel and the second microflow channel are fabricated by machining over machine tool, laser ablation, 3D printing, injection molding, or chemical etching machining.

17. The microfluidic chip-equipped detection cassette according to claim 10, wherein
the first housing is provided with a USB port, and a progress of a detection reaction is controlled by connecting or disconnecting a UBS line or controlled by turning on or turning off a power switch.

18. The microfluidic chip-equipped detection cassette according to claim 17, wherein
the temperature sensor is an NTC sensor, the temperature control module supplies power to the heating module by a built-in battery or the USB port, wherein upon power on, an internal indicator is lit and the heating module starts operating and generating heat, and when the NTC sensor senses a temperature desired for the reaction, a program enters heat preservation, and a biochemical reaction is carried out in the functional chamber.
